# EUROPEAN PATENT SPECIFICATION

(11) **EP 0 607 642 B1**
(45) Date of publication and mention of the grant of the patent: **27.09.2000**
(21) Application number: 93300324.6
(22) Date of filing: 19.01.1993
(51) Int. Cl.: A61K 7/48, A61K 7/42, A61K 31/375

(54) **Compositions for topical application to the skin for treatment and/or prevention of radiation-induced skin damage**
Mittel zur topischen Anwendung auf der Haut zur Behandlung und/oder zur Verhütung von, durch Strahlen verursachten Hautschäden
Compositions pour application topique sur la peau pour le traitement et/ou la prévention des dommages occasionnés par des radiations

(43) Date of publication of application: 27.07.1994
(73) Proprietor: PERRICONE, Nicholas V., Dr., Guilford, Connecticut 06437 (US)
(72) Inventor: PERRICONE, Nicholas V., Dr., Guilford, Connecticut 06437 (US)
(74) Representative: Bankes, Stephen Charles Digby

(56) References cited:
- EP-A- 0 308 918
- WO-A-90/12572
- WO-A-92/15292
- DE-A- 2 744 976
- FR-A- 2 244 468
- FR-A- 2 666 226
- US-A- 4 818 521
- US-A- 5 078 989
- US-A- 5 122 536
- PATENT ABSTRACTS OF JAPAN vol. 10, no. 350 (C-387)(2406)
- PATENT ABSTRACTS OF JAPAN vol. 12, no. 467 (C-550)(3314)
- PATENT ABSTRACTS OF JAPAN vol. 11, no. 282 (C-446)
- PATENT ABSTRACTS OF JAPAN vol. 11, no. 202 (C-432)(2649)
- SOAP, COSMETICS, CHEMICAL SPECIALTIES vol. 68, no. 8, 1992, NEW YORK , US page 70 'natural moisturizing & repair cream'

## Description

The present invention relates to the topical application to the skin of active agents, and/or preparations containing them, for the cosmetic and therapeutic treatment and/or prevention of radiation-induced damage to the skin, and particularly for the treatment of skin which has received excessive exposure to ultraviolet radiation, as in the case of sunburn.

Ultraviolet induced burning of the skin is most commonly seen in subjects who have been excessively exposed to natural sunlight (i.e., Sunburn), but also can be seen in subjects who have been excessively exposed to ultraviolet radiation from non-sunlight (artificial) sources, as may occur in tanning booths or incident to application of ultraviolet radiation as part of a therapeutic treatment. Cutaneous burn and other forms of skin damage also can arise from excessive or prolonged exposure to other forms of radiation outside the ultraviolet spectrum.

The clinical manifestations of ultraviolet induced burn seen in acute reactions is caused by ultraviolet radiation in the range of 290 - 320 nanometers, generally designated ultraviolet B (UVB) radiation. In artificial light sources, shorter wave lengths are also responsible for producing delayed erythema. It is now known that the longer wave lengths, i.e., 320 - 400 nanometers, designated UVA, in larger doses can also cause erythema. Included in the acute reactions of solar radiation is pigmentation. This immediate pigmentation reaction is caused by the ultraviolet radiation in the range of 320 nanometers and above. There is a delayed pigmentation reaction seen a few days after exposure, and finally there is seen an increase in thickness of the epidermis a few days after exposure, which is apparently some protective mechanism against subsequent sun exposure. The chronic reactions seen in exposure to ultraviolet radiation are premature aging of the skin due to prolonged exposure to ultraviolet radiation (this is seen clinically as irregularly distributed pigmentation and thickening of the skin) and premalignant and malignant growths of the skin.

The cause of skin damage such as that induced by excessive exposure to ultraviolet radiation is postulated to be a transfer of energy from ultraviolet radiation to the skin, resulting in generation of excited oxygen species, such as singlet oxygen, the superoxide anion, and hydroxyl radicals that can damage lipid-rich membranes with the subsequent activation of the chemical mediators of inflammation. It is well known that ultraviolet B radiation releases arachadonic acid, which is quickly oxidized to a variety of biologically active metabolites, such as prostaglandins PGD2, PGE2, PGEF2. When arachadonic acid is oxidized via the cyclo-oxygenase pathway, prostaglandins create marked erythema. Arachadonic acid oxidized via the 5-lipo-oxygenase pathway produces leukotrienes, which also can cause erythema and edema. The free radicals created by ultraviolet radiation can also damage the DNA of the cells, resulting in permanent injury, premature aging, and carcinogenesis.

The clinical symptoms of ultraviolet burn are on a spectrum from mild increased sensitivity of the skin to severe pain. It should also be noted that damage to skin can be caused by other forms of radiation, that is, ionizing radiation as well as longer wave length radiation such as infrared, which can result in erythema and pigmentation as well as premature skin ageing and malignancy. These other forms of radiation create damage by the same mechanism, i.e., generation of free radicals with subsequent damage to the cell membrane and DNA.

Suggestions for dealing with sunburn and other forms of ultraviolet radiation burn have predominantly been aimed at prevention through use of topical compositions containing agents for absorbing radiation, e.g., as exemplified by commercial sunscreen products. More recently, attention has been directed to agents which address the underlying processes involved in radiation-induced skin damage, such as the earlier-noted free radical generation processes. In this regard, investigations have been made with respect to the potential utility of ascorbic acid (Vitamin C) in the treatment and/or prevention of radiation-induced skin damage. While evidence exists that ascorbic acid has a degree of utility in this regard, commercially practical exploitation of such findings has been exceedingly difficult by reason of inability to deliver the agent to the skin in active form and difficulty in providing the agent in stable form in product preparations.

DE-A-2744976 discloses a preparation for topical application for the treatment of freckles, age spots and other abnormal pigmentation comprising as an active ingredient 4 to 25 weight % of L-ascorbic acid, which may be in the form of a derivative such as the laurate or palmitate. US-A-5078989 discloses a similar preparation, also as a skin whitening cosmetic, as does JP-A-6226210. FR-A-2244468 discloses a composition for systemic prevention of cancer, such as that induced by exposure to radiation, comprising *inter alia* ascorbic acid, which may be in the form of the laurate.

US-A-5122536 discloses a composition comprising ascorbic acid or a precursor or derivative thereof such as ascorbyl palmitate, for topical application in the treatment of psoriasis.

FR-A-2666226 discloses a skin protection cream comprising a minor amount, such as 0.05 or 0.1 weight %, of ascorbyl palmitate. A skin cream containing similar amounts of L-ascorbyl palmitate is disclosed in EP-A-308918. This composition also contains kojic acid or an ester thereof, and is used to inhibit the synthesis of melanin.

JP-A-6281307 discloses a cosmetic moisturizing and anti-wrinkle skin cream comprising 0.01 to 10 weight % of a vitamin C-γ-linoleic acid ester.

The present invention consists in the use of one or more fat-soluble fatty acid esters of ascorbic acid in the preparation of a medicament for topical application to skin areas for treating or preventing radiation-induced skin damage, by solubilizing in lipid-rich layers of the skin and scavenging therefrom free oxygen-containing radicals, neutralizing other reactive oxidants released extra-cellularly and intracellularly, and either interfering with or minimizing oxidative generation of metabolites via lipoxygenase pathways, said ester of ascorbic acid being incorporated into the medicament in an amount of at least 0.5% by weight.

In accordance with the present invention, ascorbic acid (Vitamin C), in the form of its fat-soluble fatty acid esters (e.g., ascorbyl palmitate, ascorbyl laurate, ascorbyl myristate, ascorbyl stearate and the like), and preferably in association with a dermatologically acceptable carrier in which it is dispersed or solubilized, is topically applied in effective amounts to skin areas which have been damaged, or which are susceptible to damage, by reason of radiation (especially ultraviolet)-induced mechanism.

It has been found in accordance with the invention that ascorbic acid, in the form of its fat-soluble fatty acid esters, is enormously effective in the treatment and/or prevention of radiation-induced skin damage; can be delivered, either alone or in association with a carrier, into lipid-rich skin layers without loss of efficacy; can be employed without adverse side effects; is stable against oxidation when exposed to air, a property which can be enhanced with use of a carrier for the ascorbyl fatty acid ester; and which can be employed in a wide variety of carriers either to enhance overall effectiveness or percutaneous delivery or stability. In addition, synergistic activity often noted in the use of Vitamin C along with fat-soluble vitamins such as Vitamin E or its derivatives, which synergism traditionally acts only at aqueous-lipid interfaces, can be further enhanced in accordance with the invention by reason of extension of that activity beyond water-lipid interfaces and into wholly or predominantly lipid layers.

In the preferred practice of the invention, the ascorbyl fatty acid ester is applied in admixture with a dermatologically acceptable carrier or vehicle (e.g., as a lotion, cream, ointment, soap, or the like) so as to facilitate topical application and, in some cases, provide additional therapeutic effects as might be brought about, e.g., by moisturizing of the affected skin areas. In this way it is also possible to provide preparations which can be applied in a regimen which provides a suitable and controlled dosage of the ascorbyl fatty acid ester with each application.

The amount of the ascorbyl fatty acid ester necessary to bring about the therapeutic treatment and/or prevention of the radiation-induced skin disorder is not fixed per se, and necessarily is dependent upon the severity and extent (or potential therefor) of the skin damage, the particular ascorbyl fatty acid ester employed, and the concentration of the ascorbyl fatty acid ester when employed in association with a carrier. Generally, the ascorbyl fatty acid ester or composition containing it is topically applied to the affected or implicated skin areas in a predetermined or as-needed regimen to afford protection or remedy, it generally being the case that gradual increase in such abilities is noted with each successive application. Insofar as has been determined based upon clinical studies to date, no adverse side effects are encountered.

When a carrier is employed, it is necessary that the carrier be inert in the sense of not bringing about a deactivation of the ascorbyl fatty acid ester, and in the sense of not bringing about any adverse effect on the skin areas to which it is applied.

Suitable carriers include water, alcohols, oils and the like, chosen for their ability to dissolve or disperse the active ingredient at concentrations of active ingredient most suitable for use in the therapeutic treatment or prevention. Generally, even low concentrations of active ingredient in a carrier would be suitable, requiring only that more frequent topical application be resorted to. As a practical matter, however, to avoid the need for repeated application, the topically applied composition (i.e., ascorbyl fatty acid ester plus carrier) is formulated to contain at least 0.5% by weight, more preferably at least 2 % by weight, and most preferably at least 10 % by weight, of the active ingredient, and accordingly, carriers will be chosen which can solubilize or disperse the active ingredient at such concentrations.

While the carrier for the ascorbyl fatty acid ester can consist of a relatively simple solvent or dispersant such as water or oils, it is generally preferred that the carrier comprise a composition more conducive to topical application, and particularly one which will form a film or layer on the skin to which it is applied so as to localize the application and provide some resistance to washing off by immersion in water or by perspiration and/or one which aids in percutaneous delivery and penetration of the ascorbyl fatty acid ester into lipid layers. Many such compositions are known in the art, and can take the form of lotions, creams, gels or even solid compositions (e.g., stick-form preparations). Typical compositions include lotions containing water and/or alcohols and emollients such as hydrocarbon oils and waxes, silicone oils, vegetable, animal or marine fats or oils, glyceride derivatives, fatty acids or fatty acid esters or alcohols or alcohol ethers, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophilic colloids. Such compositions are referred to herein as dermatologically acceptable carriers. Most preferred are those carriers which are fat-soluble, i.e., those which can effectively penetrate skin layers and deliver the active ascorbyl fatty acid ester to the lipid-rich layers of the skin.

In order to demonstrate the efficacy of ascorbyl fatty acid esters in treatment of radiation skin burn, a composition was prepared in which ascorbyl palmitate was mixed with a cream which contained 10,000 mg of ascorbyl palmitate per 30 g of cream. The emollient cream included the components of Stearic acid, Isopropyl myristate, Polyoxyl 40 Stearate, Stearyl alcohol, Xanthan gum, Sorbic acid, Butylated hydroxytoluene, purified water, and Diazolidinyl urea. The test subjects were five Caucasians, three women and two men. Their backs were covered with paper, with two windows 3cm by 3cm exposing skin on the left and right mid back. They were then irradiated using a high pressure metal halide lamp with broad spectrum UVB - UVA emission. Three hours after irradiation, obvious erythema appeared on the back at the two exposed sites. At that time, the subjects were asked to begin application of cream to these areas every four hours during their waking time. They were given two jars of cream, one marked "L" meaning left side of back, one marked "R" designating right side of back. They were asked to apply these appropriately to the erythematous lesions. One jar contained the emollient cream with the ascorbyl palmitate. The other jar contained the emollient cream without active ingredient. The subjects were instructed to dispense approximately one gram of cream per application. The subjects were then observed by a dermatologist at 24 hrs. and then daily for a total of three days. At the follow-up observation by the dermatologist rated at 24 hrs., a 50% reduction in erythema was noted on the side utilizing the cream with the ascorbyl palmitate content. At 48 hrs., the erythema observed on the side using the active ingredient had faded to almost imperceptible pinkness, while the side treated with the base only still had marked erythema and edema. The subjects all reported marked reduction in symptoms of burn on the side treated with active ingredient.

The effectiveness of the ascorbyl fatty acid esters in the treatment and/or prevention of radiation-induced skin damage can be postulated as resulting from the anti-oxidant properties of ascorbic acid per se, which properties are unexpectedly retained and provided to a high degree in the ascorbyl fatty acid ester form, together with the fact that the ascorbyl fatty acid ester form is capable of being delivered in an extremely effective manner. Thus, when solubilized in the lipid-rich layers of the skin, the fatty acid ester form of ascorbic acid is capable of scavenging free oxygen-containing radicals, neutralizing other reactive oxidants released extra-cellularly and intracellularly, and either interfering with or minimizing oxidative generation of metabolites via lipoxygenase pathways.

In particular, a possible explanation for the effectiveness of ascorbic acid per se in treatment of radiation damage to the skin begins by noting that ascorbic acid, which is classified as a vitamin, is a necessary nutrient in the human body, and is thus found in various concentrations in all tissues. Thus, Vitamin C plays a role in prevention of destruction of tissues by free radicals generated in normal metabolism. Ascorbic acid, or Vitamin C, is an essential vitamin, in that it prevents the disease scurvy. This is due to the fact that ascorbic acid is a co-factor in the biosynthesis of collagen.

Ascorbic acid is an anti-oxidant which can scavenge free radicals such as the oxygen radicals created by exposure of cells to radiation, as well as the generation of free radicals produced by normal metabolism. Vitamin C acts as a free radical scavenger, scavenging oxygen radicals to prevent damage to cells, as well as to stop the propagation of other free radicals. Thus, Vitamin C is categorized as a chain-breaking anti-oxidant. In addition to the fact that ascorbic acid is a powerful reducing agent, that is, preventing oxidative damage, Vitamin C also has the potential to affect the activity of prostaglandin production. It has been shown that ascorbic acid can increase cyclo-oxygenase activity in human cells. Cyclo-oxygenase is one of the pathways for the oxidation of arachadonic acid, which leads to the formation of prostaglandins which in turn mediate inflammation.

The mode of action of topical ascorbic acid in the treatment of radiation burn is likely related to the fact that ascorbic acid is a ketolactone, an extremely powerful reducing agent. Thus, an anti-oxidant such as ascorbic acid can neutralize reactive oxidants that are released extracellularly and intracellularly by exposure to radiation. Vitamin C and other biologic anti-oxidant systems are overwhelmed during the excessive activation of these oxidants by free radicals created during the absorption of energy from radiation exposure. Thus, a large supplemental amount of Vitamin C is needed locally to exert its action, and at least in theory could be supplied via topical application in amounts effective to rescue the injured cells from this hostile environment.

Use of the ascorbic acid in the form of its fatty acid ester (e.g., ascorbyl palmitate, ascorbyl laurate, ascorbyl myristate, ascorbyl stearate) is greatly beneficial for a variety of reasons. In particular, in this form the fatty acid ester is better able to penetrate the skin, allowing it to be released in a fat-soluble environment. Also, in this form the fatty acid ester can be solubilized in a fat-soluble base (cream, lotion, etc.) which aids in skin penetration. Still further, this fatty acid ester form of ascorbic acid is less prone to oxidative breakdown (and particularly so when further admixed in a fat-soluble base), and thus is capable of being made up as a stable preparation having a suitably long shelf-life and which has prolonged effectiveness after being applied to the skin.

Generally speaking, the present invention is one which involves the topical application of fat-soluble fatty acid esters of ascorbic acid as a means for bringing about and/or augmenting any or all of the effects or actions which ascorbic acid normally brings about in vivo, i.e., its role in the synthesis of collagen, its role as a free radical scavenger or neutralizer, its role as an inhibitor of the lipoxygenase oxidation pathway (i.e., as a result of being a cyclo-oxygenase agonist), and the like, with consequent efficacy in the treatment and/or prevention of radiation-induced skin damage. By virtue of the fat-solubility of these fatty acid esters and the further enhancement of this solubility via admixture with fat-penetrating carriers, the active ascorbic acid can be effectively percutaneously delivered to lipid layers so as to bring about these effects and actions, and further can be utilized in forms which protect it against oxidative loss of activity.

The method of the present invention is particularly useful for the treatment of skin which has been damaged by exposure to ultraviolet radiation (and for preventing such damage) but, based upon the likely mechanism of action, also is useful in general for treatment or prevention of any radiation-induced skin damage, particularly that associated with free radical related damage. As such, the topical application of fatty acid esters of ascorbic acid according to the invention can also be effective for chronic administration to prevent the free radical damage seen in the natural aging process of the skin and the free radical damage caused by chronic exposure to sunlight. The fatty acid esters of ascorbic acid can thus be conveniently added to commercial sun-screens to enhance their anti-aging and anti-cancer activity. It can also be applied prior to sun exposure for added protection against burn.

## Claims

1. The use of one or more fat-soluble fatty acid esters of ascorbic acid in the preparation of a medicament for topical application to skin areas for treating or preventing radiation-induced skin damage, by solubilizing in lipid-rich layers of the skin and scavenging therefrom free oxygen-containing radicals, neutralizing other reactive oxidants released extra-cellularly and intracellularly, and either interfering with or minimizing oxidative generation of metabolites *via* lipoxygenase pathways, said ester of ascorbic acid being incorporated into the medicament in an amount of at least 0.5% by weight.

2. Use according to claim 1 wherein said fat-soluble fatty acid ester of ascorbic acid is ascorbyl palmitate, ascorbyl laurate, ascorbyl myristate, ascorbyl stearate or a mixture of two or more thereof.

3. Use according to claim 2 wherein said fat-soluble fatty acid ester of ascorbic acid is ascorbyl palmitate.

4. Use according to any preceding claim wherein said fat-soluble fatty acid ester of ascorbic acid is incorporated in an amount of at least 2.0% by weight.

5. Use according to claim 5 wherein said fat-soluble fatty acid ester of ascorbic acid is incorporated in the medicaments in an amount of at least 10% by weight.

6. Use according to any preceding claim wherein vitamin E is incorporated into the medicament.

7. Use according to any preceding claim wherein the fatty acid ester of ascorbic acid is incorporated into a dermatologically acceptable carrier for topical application.

## Patentansprüche

1. Verwendung von einem oder mehreren fettlöslichen Fettsäureestern von Ascorbinsäure bei der Herstellung eines Medikamentes für die Oberflächenanwendung auf Hautbereichen zur Behandlung oder Vorbeugung von strahleninduziertem Hautschaden, durch Solubilisieren und Spülen von freien sauerstoffhaltigen Radikalen in/aus lipidreichen Schichten der Haut, Neutralisieren anderer reaktiver Oxydationsmittel, die extrazellulär oder intrazellulär freigesetzt wurden, und entweder Stören oder Minimieren einer oxydativen Generierung von Metaboliten über Lipoxygenase-Wege, wobei das genannte Ester der Ascorbinsäure in das Medikament in einer Menge von wenigstens 0,5 Gew.-% eingebaut wird.

2. Verwendung nach Anspruch 1, bei der das genannte fettlösliche Fettsäureester von Ascorbinsäure Ascorbylpalmitat, Ascorbyllaurat, Ascorbylmyristat, Ascorbylstearat oder ein Gemisch aus zwei oder mehreren davon ist.

3. Verwendung nach Anspruch 2, bei der das genannte fettlösliche Fettsäureester von Ascorbinsäure Ascorbylpalmitat ist.

4. Verwendung nach einem der vorherigen Ansprüche, bei der das genannte fettlösliche Fettsäureester von Ascorbinsäure in einer Menge von wenigstens 2,0 Gew.-% eingebaut wird.

5. Verwendung nach Anspruch 4, bei der das genannte fettlösliche Fettsäureester von Ascorbinsäure in die Medikamente in einer Menge von wenigstens 10 Gew.-% eingebaut wird.

6. Verwendung nach einem der vorherigen Ansprüche, bei der Vitamin E in das Medikament eingebaut wird.

7. Verwendung nach einem der vorherigen Ansprüche, bei der das Fettsäureester von Ascorbinsäure in einen dermatologisch akzeptablen Träger für Oberflächenanwendung eingebaut wird.

## Revendications

1. L'utilisation d'un ou plusieurs esters d'acide gras de l'acide ascorbique, solubles dans la graisse, pour la préparation d'un médicament pour application locale à des zones cutanées pour traiter ou prévenir des lésions de la peau dues aux rayonnements, en solubilisant les couches riches en lipides de la peau et en les débarrassant de radicaux libres contenant de l'oxygène, en neutralisant d'autres oxydants réactifs libérés extra-cellulairement et intra-cellulairement, et soit en perturbant soit en minimisant la génération oxydante de métabolites par l'intermédiaire des voies de la lipoxygénase, ledit ester de l'acide ascorbique étant incorporé dans le médicament en une quantité minimum de 0,5% en poids.

2. Usage selon la revendication 1, selon lequel ledit ester d'acide gras de l'acide ascorbique soluble dans la graisse est le palmitate d'ascorbyle, le laurate d'ascorbyle, le myristate d'ascorbyle, le stéarate d'ascorbyle ou un mélange de deux de ceux-ci ou davantage.

3. Usage selon la revendication 2, selon lequel l'ester d'acide gras de l'acide ascorbique soluble dans la graisse est le palmitate d'ascorbyle.

4. Usage selon l'une quelconque des revendications précédentes, selon lequel l'ester d'acide gras de l'acide ascorbique soluble dans la graisse est incorporé en une quantité minimum de 2,0% en poids.

5. Usage selon la revendication 5, selon lequel l'ester d'acide gras de l'acide ascorbique soluble dans la graisse est incorporé dans les médicaments en une quantité minimum de 10% en poids.

6. Usage selon l'une quelconque des revendications précédentes, selon lequel de la vitamine E est incorporée dans le médicament.

7. Usage selon l'une quelconque des revendications précédentes, selon lequel l'ester d'acide gras de l'acide ascorbique est incorporé dans un véhicule admissible en dermatologie pour l'application locale.
